# EUROPEAN PATENT APPLICATION

(11) **EP 4 066 879 A1**
(43) Date of publication of application: **05.10.2022**
(21) Application number: 20891822.7
(22) Date of filing: 25.11.2020
(51) Int. Cl.: A61M 31/00, A61M 1/00, A61M 3/02

(54) **DUAL-BODY DRUG ADMINISTRATION DEVICE HAVING FUNCTION OF REMOVING MUCUS FROM PARANASAL SINUS**

(30) Priority: 25.11.2019 KR 20190152049; 28.08.2020 KR 20200109190
(71) Applicant: Fluchem Ltd, Daejeon 34078 (KR)
(72) Inventor: KIM, Dae Whang, Daejeon 34083 (KR); KIM, Seung Shin, Seoul 05658 (KR); PARK, Yang Ok, Daejeon 34083 (KR); KIM, Jung Wook, Daejeon 34083 (KR)
(74) Representative: Gerauer, Marc Philippé
(86) International application number: PCT/KR2020/016876
(87) International publication number: WO 2021/107616

(57) **Abstract**

The present invention relates to a dual-body drug administration device which administers drugs and removes mucus from the paranasal sinus through the nostrils in order to prevent or treat a paranasal sinus disease and, more specifically, to a device in which two injectors having adapters coupled thereto are integrally fixed by a fixing holder, the adapters of the fixed injectors are simultaneously brought into contact with both the nostrils, and then negative pressure is generated in the nasal cavity to pull out mucus from the paranasal sinus or administer a drug to the paranasal sinus.

## Description

### Technical Field

The disclosure relates to a dual-body drug administration device for removing mucus from a paranasal sinus through a nostril and administrating drug therein in order to treat a paranasal sinus disease and, more specifically, to a device for integrally fixing two injectors having adapters coupled thereto by a fixing holder, adjusting the adapter interval such that the two fixed injectors are forced against a user's both nostrils, respectively, and generating a negative pressure in the nasal cavity such that mucus is discharged from a paranasal sinus, or drug is administered into the paranasal sinus.

### Background Art

Paranasal sinuses refer to air-filled spaces inside the skull, behind the forehead, and behind the nasal bone, cheeks, and eyes. When filled with air, the paranasal sinuses vibrate and help speech. Four pairs of paranasal sinuses are connected to the nasal cavity through small holes.

When a person is healthy, ciliary actions freely discharge mucus from paranasal sinuses, thereby maintaining the inside clean. The mucus is a thin liquid full of moisture, and freely flows out from paranasal sinuses to the upper nose and is discharged to the nose entrance and back neck. Mucus discharge maintains the nose moist and washes away dust, bacteria, and other microorganisms.

However, if inflammation occurs in paranasal sinuses, the mucus becomes thick and sticky, and thus cannot flow through ostia leading to the nose. Body fluids accumulate in the paranasal sinuses and causer pressure and pain, thereby resulting in paranasal sinusitis. The definition of paranasal sinusitis is essentially identical to paranasal sinus infection.

If air pockets in paranasal sinuses are blocked, viruses, bacteria, mold, or other microorganisms multiply. If upper respiratory infection (for example, common cold or influenza), allergy, or clogging blocks natural discharge of mucus, the accumulated mucus provides a perfect place for bacteria multiplication and inflammation. Thick, yellow, or discolored mucus is a typical example of such infection.

Paranasal sinusitis caused by such an infection has two types (acute and chronic). Acute paranasal sinusitis lasts from ten days to eight weeks, and chronic paranasal sinusitis lasts longer than the acute paranasal sinusitis.

Chronic paranasal sinusitis, once occurred, may last multiple months, and mold infection typically lasts for a long time. Acute paranasal sinusitis has a general problem involving facial pain and pressure, and may cause skin flare above paranasal sinuses, nasal obstruction, catarrh, heat, and headache.

If paranasal sinusitis occurs, antibiotics, nasal hyperemia removing agents, antihistamines, nasal corticosteroids, nasal saline lavage, or other methods and drugs are used to treat the same. Acute paranasal sinusitis is commonly treated by using antibiotics and decongestants. It is recommended, in order to treat chronic paranasal sinusitis, to rest and to drink sufficient water such that mucus becomes thin. Antibiotic treatment takes effects only after a long period of time (about 8-12 weeks), which has a high probability of side effects, and thus is not recommended in most cases. In addition, if the paranasal sinusitis persists for three months or longer even after using antibiotics and antihistamines continuously, a surgery is recommended in the last resort.

The most commonly practiced surgery nowadays is functional endoscopic sinus surgery (FESS). The goal of FESS is to widen openings of the maxillary sinus and frontal sinus and to remove ethmoidal cells while manually watching the same such that, by opening the ethmoidal sinus area, liquid discharge from the paranasal sinusitis is improved.

However, FESS itself easily causes inflammation and may cause post-surgery fibrosis, stenosis, and/or polyposis. As a result, newly-opened paranasal sinuses are frequently closed, and additional surgeries may be necessary to correct openings and to main opened areas.

As described above, paranasal sinus surgeries are risky, and drug treatment, without surgery, is recommended in most cases.

Paranasal sinusitis locally occurs in the nasal cavity and paranasal sinuses, it may be more effective to administer medication locally in the inflammation-affected area than to administer medication systemically. Particularly, local drug delivery is advantageous in that systemic side effects can be avoided because drug directly affects the infected area, and the target area can be treated with a higher density.

Various types of devices and methods have been proposed to locally administer medication for paranasal sinusitis treatment, such as a non-cleaning device, a spray pump device, a nasal drop device, and an atomizer device.

Experiments by Snidvongs (Am J Rhinol. 2008 Sep-Oct; 22(5): 483-6) et al. confirm that a very small amount of drug enters paranasal sinuses of a patient having inflammation. This is the major reason drug for paranasal sinus treatment has no effect.

Korean Registered Patent No. 10-1947403 (date of registration: February 7, 2019; hereinafter, referred to as "Prior Document 1") discloses a device and a method for administering drug into the naval cavity and paranasal sinuses. According to Prior Document 1, a variable-volume adapter is mounted to the inlet of a drug storage device such that the adapter inner volume is changed by pressurizing and depressurizing the adapter, and a pressure change occurs in the nasal cavity and paranasal sinuses by delivering the amount of volume change, thereby washing the nasal cavity and paranasal sinuses and administering drug therein. However, Prior Document 1 is not appropriate for removing secretion from paranasal sinuses, although drug can be effectively injected into paranasal sinuses.

Korean Registered Patent No. 10-1627610 (date of registration: May 31, 2016; hereinafter, referred to as "Prior Document 2") discloses a nasal washing device. The device in Prior Document 2 includes one body portion and two nozzle portions such that saline can be simultaneously injected through both nostrils to wash them. However, the device has difficulty in washing paranasal sinuses by means of pressure change, and the two nozzle portions have the same action. In other words, it is difficult to provide different functions with respective nozzle, for example, drug injection with one, and secretion absorption with the other.

Japanese Laid-open Patent Publication No. H9-253208 (date of publication: September 30, 1997; hereinafter, referred to as "Prior Document 3") discloses a nasal administration device. Prior Document 3 has a structure similar to that of Prior Document 2 in that a single body has two spraying openings formed therein. This device can similarly spray uniform drug into both nostrils, but is not appropriate for suctioning/removing secretion in paranasal sinuses by using a negative pressure.

As described above, in order to effectively treat paranasal sinusitis, mucus needs to be removed from paranasal sinuses before administering drug. However, it is currently difficult to remove mucus from paranasal sinuses without a surgical procedure, and the maxillary sinus, among the paranasal sinuses, has a discharge hole in a high position. As a result, it is difficult to discharge secretion by gravity, the same is thus easily infected, and infectants are very difficult to remove.

That is, it is crucial to remove mucus from paranasal sinuses in order to treat paranasal sinusitis, but there is currently no method for removing mucus from paranasal sinuses except for expensive surgical methods.

Therefore, there have been gradually increasing interests and needs regarding a technology for effectively removing mucus from paranasal sinuses at a low treatment cost without a surgical procedure.

### Disclosure of Invention

### Technical Problem

In order to solve the above-mentioned problem related to the need to remove mucus from paranasal sinuses, the disclosure provides a dual-body drug administration device having a function for removing mucus from paranasal sinuses, wherein
two injectors having adapters are used while being forced into both nostrils; when the head is inclined laterally, the two injectors are arranged one above the other, thereby sealing the nasal cavity; injector push rods are pulled while nasal breathing is blocked by lifting the soft palate, thereby forming a negative pressure in the nasal cavity; the negative pressure formation discharges secretion from the upper paranasal sinus to the upper nasal cavity, or the negative pressure is released such that drug is injected from the lower nasal cavity into the lower paranasal sinus; secretion introduced into the upper nasal cavity in the process of drug injection into the lower paranasal sinus can also be remove instantly; and drug injection and secretion suction/removal can be performed simultaneously, as described above, or secretion removal and drug injection can be performed respectively.

Particularly, it is an aspect of the disclosure to provide a device wherein a fixing holder is used to integrally connect two conventional single nasal washing devices, the interval between adapter parts used to inject drug and to suction secretion can be easily adjusted, and the same thus can closely adhere to the user's nostrils, thereby improving the efficiency of paranasal sinus mucus removal or drug administration.

### Solution to Problem

A dual-body drug administration device having a function for removing mucus from paranasal sinuses according to the disclosure, for solving the above-mentioned problems, is
a dual-body drug administration device for injecting a drug into a nasal cavity through a nostril, the dual-body drug administration device including: a) a first injector having a coupling element, which is formed at the front end thereof and to which a first adapter is coupled, and a space part formed therein; b) a second injector having a coupling element, which is formed at the front end thereof and to which a second adapter is coupled, and a space part formed therein; and c) a fixing holder configured to mount and fix the first injector and the second injector such that a distance between centers of an inlet/outlet of the first adapter and an inlet/outlet of the second adapter is 0.5 cm to 10 cm.

In addition, the fixing holder may be configured to: allow the first injector and the second injector to be mounted in parallel so as to be in close contact with user's both nostrils; or allow the first injector and the second injector to be mounted at an acute angle in a slant state in which an interval between the rear sides thereof is wider than that between the front sides thereof so that an interval between the inlets/outlets of the first adapter and the second adapter is adjusted to be brought into close contact with and close the user's both nostrils.

In addition, the fixing holder may be provided in a case form configured to cover to receive bodies of the first injector and the second injector, or in a clip form to which the first injector and the second injector are inserted into opposite lateral sides to be fixed.

In addition, the fixing holder may be provided in a case form so as to include: a holder body having mounting grooves configured to allow the first injector and the second injector mounted thereto, respectively; and a holder cover configured to hold and fix the mounted first injector and second injector by covering the mounting grooves of the holder body.

The fixing holder may further include interval adjustment parts formed between two mounting grooves of the holder body, the interval adjustment parts formed between the mounting grooves being configured to be separate; on the front side, the separate interval adjustment parts are rotatably connected by a hinge pin; and in the rear side, an arc-shaped guide hole having multiple catching protrusions formed therein is formed through one interval adjustment part side and a catching bar protrudes upward from the other interval adjustment part side, such that an angle between two mounting grooves is adjusted by overlapping the catching bar to be inserted into the art shaped guide hole and left/right rotation.

Furthermore, the fixing holder may include: a clip body having mounting grooves configured to allow the first injector and the second injector mounted thereto; and clip parts respectively protruding toward both lateral sides from both ends in the axial direction of the clip body and configured to hold each of the front end and rear end of the first injector and the second injector to be prevented from deviating toward the lateral sides.

In addition, the fixing holder may further include: in the clip part of the front end of the clip body, a catching projection protruding in a direction of the center axis of the mounting grooves to prevent the mounted first injector and second injector from being pushed to the front by catching the front ends thereof; and in the clip part of the rear end of the clip body, an insertion groove formed in a direction perpendicular to the center axis of the mounting grooves to allow handle parts formed at the rear ends of the first injector and the second injector to be inserted and fitted thereto.

The depth of the insertion groove may be formed deeper toward the inside from a surface of the mounting grooves to allow a protruding handle part of an injector mounted to the mounting grooves to axially rotate without interruption.

In addition, the clip body may be configured to be separated into a front body and a rear body to allow length adjustment.

In the front body, a length adjustment groove may be formed from the rear end toward the front side and a coupling hole may be formed in a lateral direction orthogonal to the length adjustment groove to be coupled to allow a coupling pin to penetrate therethrough, and on the rear body, a length adjustment bar having a section corresponding to a section of the length adjustment groove may protrude from the front end in the forward direction to be inserted into the length adjustment groove, a long coupling hole may be formed inside the length adjustment bar to penetrate in a lateral direction and elongated in the forward/backward direction to allow the coupling pin to penetrate therethrough so that a length is adjusted according to a coupling position of the long coupling hole and the coupling pin.

In addition, a negative pressure may be generated in the nasal cavity by forward/backward movement of a push rod of the first injector or the second injector to allow mucus inside the paranasal sinus to be discharged or a drug to be injected into the paranasal sinus.

In addition, a fixing holder as a device included in the dual-body drug administration device may allow two injectors to be mounted and fixed thereto.

In addition, an adapter, as a device included in the dual-body drug administration device, may have a communication groove formed at the end surface of an inlet/outlet thereof in a lateral direction such that internal and external portions communicate with each other.

### Advantageous Effects of Invention

The disclosure provides, as a solution to the above-mentioned problems, a dual-body drug administration device having a function for removing mucus from paranasal sinuses, wherein
the device includes two injectors which are connected by a fixing holder for fixing the same and are thereby integrated such that the user's two nostrils are simultaneously blocked, thereby sealing the insides of the two nostrils; the piston push rod of one injector is moved forwards/backwards so as to change the pressure inside the nasal cavity; and secretion discharged from paranasal sinuses to the nasal cavity by the pressure change is suctioned/discharged through the injector. In addition, if the nasal cavity pressure is changed while drug remains injected into the nasal cavity, the drug may be easily moved from the nasal cavity into paranal sinuses by the pressure change. This may maximize the effect of the drug by moving the drug from the nasal cavity into the paranasal sinuses.

In addition, the two injectors are integrally fixed by the fixing holder such that the interval between injection openings or suction openings at front ends of the injectors can be adjusted according to the interval between the user's nostrils, thereby facilitating nasal cavity closure inside the nostrils.

In addition, concurrently with nasal cavity closure, secretion removal or drug injection may be conducted by a pressure change through the nasal cavity on one side, or secretion removal and drug injection may be simultaneously conducted by a pressure change through the nasal cavity on both sides, thereby improving use convenience.

In addition, by injecting drug into paranasal sinuses through the nasal cavity, it is possible to prevent and treat diseases occurring in the nasal cavity and paranasal sinuses due to various respiratory infection (for example, common cold, influenza, rhinitis), and to effectively alleviate or eliminate symptoms within a short period of time.

### Brief Description of Drawings

FIG. 1A and FIG. 1B are a perspective view and a horizontal sectional view illustrating a dual body drug administration device according to a preferred embodiment of the disclosure.
FIG. 2A is a sectional view illustrating a separable adapter according to an embodiment of the disclosure, FIG. 2B is a sectional view illustrating an adapter having a V-shaped communication groove formed at a front end of an inlet/outlet of the adapter, FIG. 2C is a sectional view illustrating another form of an adapter according to the disclosure.
FIG. 3A to FIG. 3C are vertical sectional views illustrating various shapes of a fixing holder of the disclosure.
FIG. 4A to FIG. 4B are planar views illustrating a fixing holder including an interval adjustment part formed thereon according to an embodiment of the disclosure.
FIG. 5A to FIG. 5D are bottom views and an exploded perspective view illustrating a fixing holder having a variable interval adjustment part according to an embodiment of the disclosure.
FIG. 6A to FIG. 6D are perspective views and a schematic view illustrating a clip-type fixing holder according to an embodiment of the disclosure.
FIG. 6E is a partial sectional view illustrating a rear side of a clip-type fixing holder according to another embodiment of the disclosure.
FIG. 6F is a sectional view taken along B-B of FIG. 6D, which schematically illustrates that a handle part of an injector is freely rotated by an insertion groove.
FIG. 6G is a schematic view illustrating a change in position of a coupling hole at the front of an injector by rotation of an injector according to an embodiment of the disclosure.
FIG. 7A to FIG. 7C are a planar view and sectional views illustrating a clip body including a length adjustment groove and a length adjustment bar according to another embodiment of the disclosure.
FIG. 8A to FIG. 8G are schematic views illustrating a process of use of a dual-body drug administration device according to the disclosure.

### Best Mode for Carrying out the Invention

Hereinafter, embodiments of the disclosure will be described in detail with reference to accompanying drawings. The disclosure may be modified in various forms, specific embodiments thereof are shown by way of example in the drawings and will herein be described in detail. It should be understood, however, that there is no intent to limit the disclosure to the particular forms disclosed, but on the contrary, the disclosure is to cover all modifications, equivalents, and alternatives falling within the spirit and technique scope of the disclosure. Like numbers refer to like elements throughout the description of the figures. In the drawings accompanied herein, the dimensions of structures are magnified or reduced compared to the actual dimensions for clarity of the disclosure.

The terminology used herein is for the purpose of describing particular embodiments only and is not intended to be limiting of the disclosure. The terms of a singular form may include plural forms unless they have a clearly different meaning in the context. It will be further understood that the terms "include", "comprise", or "have" when used in this specification, specify the presence of stated features, numbers, steps, operations, elements, or combinations thereof, but do not preclude the presence or addition of one or more other features, numbers, steps, operations, elements, or combinations thereof.

Unless defined otherwise, all technical and scientific terms used herein have the same meaning as commonly understood by a person of ordinary skill in the art to which this inventive concept belongs. It will be further understood that terms, such as those defined in commonly used dictionaries, should be interpreted as having a meaning that is consistent with their meaning in the context of the relevant art and will not be interpreted in an idealized or overly formal sense unless expressly so defined herein.

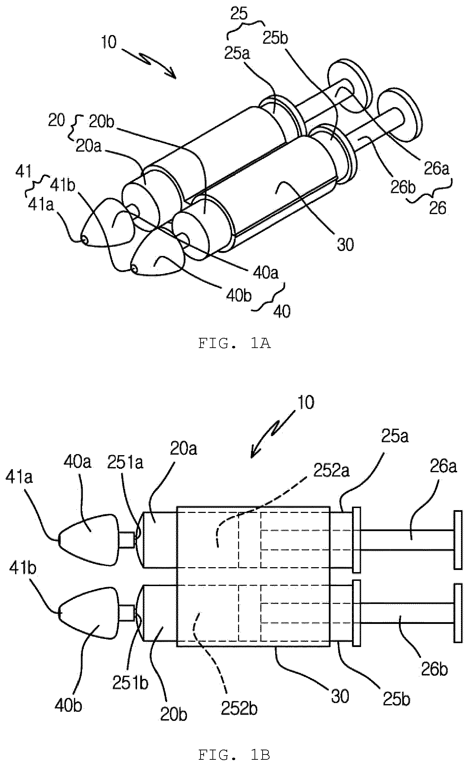

FIG. 1A and FIG. 1B are a perspective view and a cross-sectional view illustrating a dual body drug administration device according to a preferred embodiment of the disclosure.

Referring to the drawings, the dual-body drug administration device 10 according to the disclosure is formed by including two injectors 20 constituted of a first injector 20a and a second injector 20b.

The first injector 20a and the second injector 20b have a syringe form including bodies 25, 25a, 25b having space parts 252a, 252b formed therein and

piston push rods 26, 26a, 26b inserted into the space parts of the bodies and moved in an axial direction, coupling elements 251a, 251b protrude from the front of the bodies, and a first adapter 40a and a second adapter 40b are coupled to the coupling elements, respectively.

In this structure, when the piston push rods 26, 26a, 26b are moved in a forward/backward direction which is the axial direction, the volumes of the space parts between the coupling elements 251a, 251b and the piston push rods 26, 26a, 26b are decreased or increased.

When a drug is stored in the space parts 252a, 252b, the drug is discharged toward the front, and when the space parts are empty, a negative pressure may be formed in the nasal cavity by suctioning air in the nasal cavity or a secretion may be suctioned and removed.

The first injector 20a and the second injector 20b may be formed of a plastic, glass, or metal material having a volume of 5 ml to 50 ml.

The first adapter 40a and the second adapter 40b have inlets/outlets 41, 41a, 41b formed through the fronts thereof and have the rears coupled to the coupling elements 251a, 251b formed on the bodies 25, 25a, 25b of the first injector and the second injector.

In addition, the first adapter 40a and the second adapter 40b have at least one of a form in which the front protrudes to be in close contact with nostrils and have a portion thereof inserted into the nostrils and the rear has a shape of a wide heart, hemisphere, cone, or truncated cone, a form in which a triangular or polygonal container has a front end side a portion of which is formed in a cone or truncated cone, and a form in which a portion of the front end side of a polygonal container have a cone or truncated cone shape with a slant surface is formed to be convex or concave, such that close contact with the nostrils and tight sealing are achieved. The inlets/outlets 41, 41a, 41b of the adapters may have circular, square, or heterogeneous shapes.

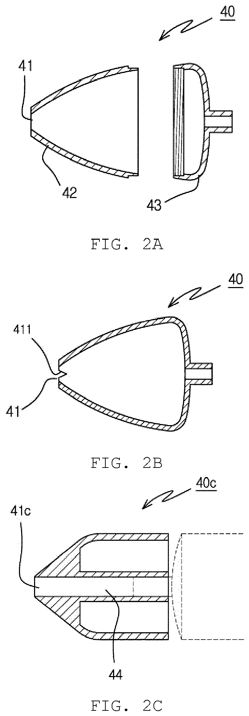

Furthermore, the adapters may include a separable adapter 40 as shown in FIG. 2A so as to facilitate internal cleaning thereof. That is, the adapters may be divided into a front adapter part 42 having the inlet/outlet 41 formed therethrough and a rear adapter part 43 coupled to the coupling element of the injectors, and the front adapter part and the rear adapter part may be provided in a structure detachably coupled to each other by various coupling methods such as screw coupling or insertion coupling.

In addition, the adapters may have a V-shaped communication groove 411 additionally formed at the front-end surface of the inlet/outlet 41 of the adapters as shown in FIG. 2B. When the adapters are brought into close contact with the nasal cavity, the communication groove 411 prevents blocking of the end part of the inlet/outlet of the adapters against the inner wall of the nasal cavity so as to facilitate communication of substances inside and outside the adapters. That is, even when the end part of the inlet/outlet 41 is blocked by the inner wall of the nasal cavity, a lateral side may be opened by the communication groove 411 formed on a surface of the inlet/outlet to allow communication of substances inside and outside so as to form a negative pressure.

The communication groove 411 may be formed in various shapes such as a U-shape in addition to the V-shape shown above. In addition, one or multiple communication grooves may be formed at the end surface of the inlet/outlet of the adapters.

As such, when sealing the nostrils, according to action of the piston push rod 26 of the first injector 20a or the second injector 20b, a drug may be injected into the sealed nasal cavity or air inside the nasal cavity is suctioned to form a negative pressure inside the nasal cavity.

The first adapter 40a and the second adapter 40b may be formed of one of plastic, silicone, rubber, or elastic plastic to enhance sealing force by improving an adhesion property to the outer surface of the nostrils.

Although, it has been described that the adapters 40, 40a, 40b are detachably attached to the injectors 20, 20a, 20b, the adapters and the injectors may be provided in an integrated form as well.

Next, the first injector 20a and the second injector 20b may be integrally formed by a fixing holder 30.

In addition, as shown in FIG. 2C, only a fluid channel 44 may be formed inside the adapter 40c without a reception space so as to directly communicate from the inlet/outlet 41c to the injector coupled the rear side thereof. That is, while a reception space is formed in the adapter described above to temporarily receive partial secretion or a drug, only the fluid channel is formed in this form and thus secretion or a drug may be directly moved from the nasal cavity to the injector, and from the injector to the nasal cavity. Here, it is preferable that the outer portion of the adapter 40c is expanded to have a conical shape so as to seal the nostrils when coming in close contact with the user nostrils.

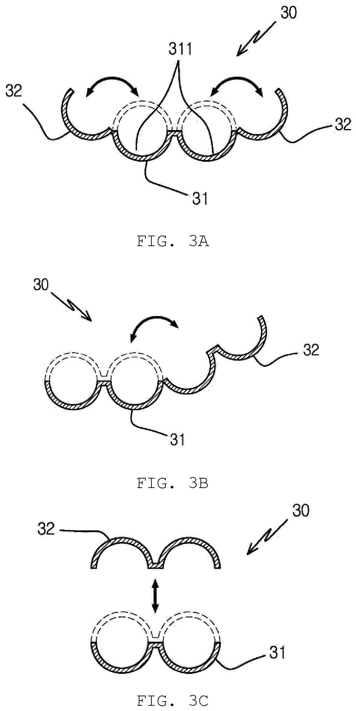

As shown in FIG. 3A, the fixing holder 30 of the disclosure includes a holder body 31 having mounting grooves 311 for allowing each of the first injector 20a and the second injector 20b to be mounted thereto, and a holder cover 32 extending from a lateral side of the holder body and rotating to cover upper parts of the first injector and the second injector mounted in the mounting grooves. Here, the outer end of the holder cover 32 is coupled to and fixed to a portion between two mounting grooves of the holder body by rotation.

A coupling means for the holder body 31 and the holder cover 32 may employ various well-known coupling means such as a catching protrusion and a catching groove, a button, a Velcro tape, and a magnet.

In addition, the structure of the holder body 31 and the holder cover 32 may be provided as a form in which holder covers 32 extend from the both lateral sides of the holder body 31 to cover injectors, respectively as shown in FIG. 3A, a form in which a holder cover 32 extends from one lateral side of the holder body 31 to cover two injector as shown in FIG. 3B, or a form in which the holder body 31 and a holder cover 32 are attached or detached while completely separated from each other as shown in FIG. 3C.

The fixing holder 30 surrounds a portion of the outer surface of the first injector and the second injector, and integrally binds the fixing holder, the first injector, and the second injector.

In addition, a coating layer for providing frictional force may be further provided on an inner surface of the mounting groove of the holder body 31 of the fixing holder 30 and an inner surface of the holder cover 32 to limit movement of the injectors mounted thereto in the axial direction. The coating layer is preferably formed of a material with an anti-slip function such as rubber. When having a length identical to that of the injector, the fixing holder 30 may have catching projections at both ends of the holder to limit the movement of the injectors in the axial direction.

In addition, it is preferable that a distance between centers of each inlet/outlet 41a, 41b of the first adapter 40a and the second adapter 40b of the first injector and the second injector fixed by the fixing holder 30 falls within 0.5 to 10 cm to facilitate insertion into both nostrils of a user.

The fixing holder 30 may be formed of one of plastic, silicone, rubber, or a metal material, and may cause the injectors to be parallel with each other or slant to each other to have an acute interior angle therebetween so that a space between the inlets/outlets 41a, 41b of the first adapter 40a and the second adapter 40b mounted to the first injector 20a and the second injector 20b is maintained.

As such, the fixing holder 30 has a fixing structure so that the first injector or the second injector is prevented from being deviated from the fixing holder when the piston rod is pushed or pulled while the first injector and the second injector remain mounted.

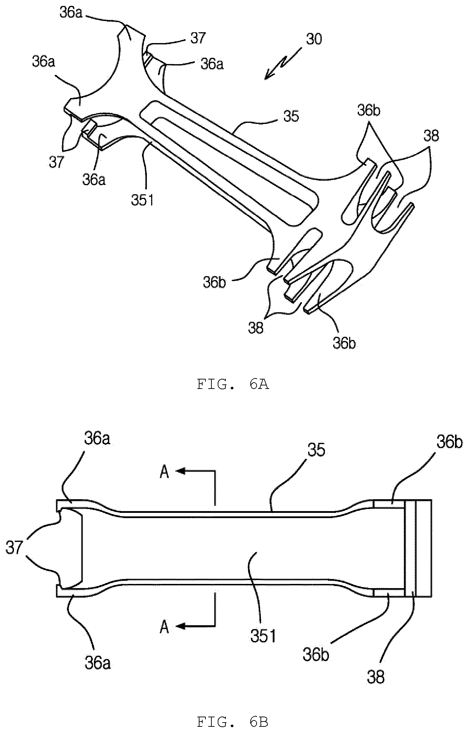

For example, as shown in the drawings, in addition to the structure in which the fixing holder 30 having a case shape is provided so that the first injector and the second injector are mounted in the holder body and then covered by the holder cover to be coupled, and the front end and the rear end of the fixing holder are caught by the front end and rear end of the first injector and second injector bodies 25, 25a, 25b, respectively, there may be provided various structures for fixing or limiting movement of the first injector and the second injector by catching or fitting the same into the fixing holder, such as a structure in which a box-type fixing holder is provided so that the first injector and the second injector are fitted into a box body in the axial direction to be fixed, a structure in which a clip type fixing holder is provided to be coupled by a clip as shown in FIG. 6A to FIG. 6D, or a combined structure thereof.

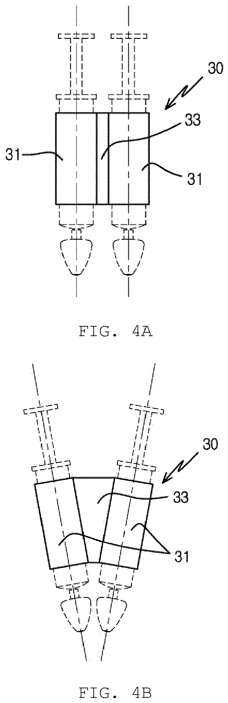

As shown in FIG. 4A and FIG. 4B, an interval adjustment part 33 is further formed between two mounting grooves in the holder body 31 to cause an interval between the mounting grooves 311 to be spaced apart by a predetermined width. The interval adjustment part 33 may be formed to have a rectangular shape to maintain axial intervals formed by two mounting grooves to be parallel to each other or the interval adjustment part may be provided in a trapezoidal shape to cause two axes of the mounting grooves to intersect with an interior angle of a predetermined angle. Here, the interior angle, which is an axial angle, forms an acute angle, preferably forms an angle of 5-45 degrees to prevent an operation from becoming inconvenient due to an excessive space of the piston push rods which correspond to the rear ends of the first injector and the second injector.

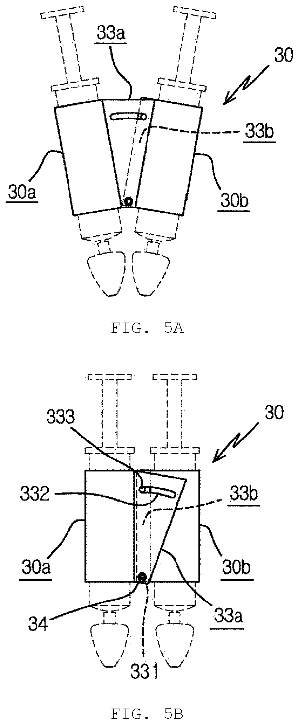

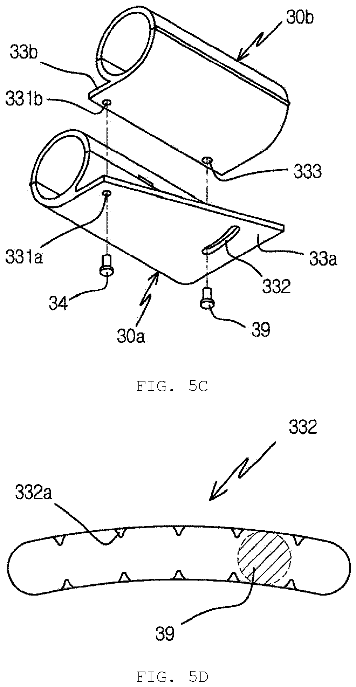

FIG. 5A and FIG. 5B are bottom views illustrating a fixing holder and FIG. 5C is an exploded perspective view. The fixing holder 30 may be configured so that the axial angle is variable by separating the interval adjustment parts 33, 33a, 33b as shown in the drawings.

Referring to the drawings, the fixing holder includes a first fixing holder 30a and a second fixing holder 30b separate from each other to form respective mounting grooves, and interval adjustment parts 33a, 33b extend from lateral sides facing each other of the first fixing holder 30a and the second fixing holder 30b, respectively while shapes of the interval adjustment parts formed on the first fixing holder and the second fixing holder are different from each other.

For example, the interval adjustment part 33a of the first fixing holder 30a extends in a narrow width toward the front and in a wide width toward the rear, a hinge hole 331a into which a hinge pin 34 is inserted and installed is formed through the front side of the inner surface of the extended interval adjustment part, and an arc-shaped guide hole 332 having the hinge hole as the center of rotation center and a long arc shape is formed through the rear side so as to allow an interval adjustment pin 39 to penetrate the same.

In addition, the interval adjustment part 33b of the second fixing holder 30b extends in a narrow width toward the front and the rear, and a hinge hole 331b and a fixing hole 333 are formed through the front side and the rear side of the extended interval adjustment part to allow a hinge pin 34 and an interval adjustment pin 39 to be inserted thereinto, respectively. Accordingly, the interval adjustment pin 39 installed by penetrating the arc-shaped guide hole 332 and the fixing hole 333 may be tightened to be fixed after adjusting an interval.

The first fixing holder 30a and the second fixing holder 30b having the configuration may arrange each hinge hole 331 in the same vertical axis while overlapping the extended interval adjustment parts 33a, 33b to be integrally coupled so as to be rotated by the hinge pin 34, and cause the interval adjustment pin 39 to penetrate the fixing hole 333 of the second fixing holder and the arc-shaped guide hole 332 of the first fixing holder so that the interval adjustment pin 39 rotates within the arc-shaped guide hole 332 around the hinge pin 34 to change an angle between center axes of the mounting grooves formed in the first fixing holder and the second fixing holder so as to cause an interval between the inlets/outlets 41a, 41b of the first adapter 40a and the second adapter 40b to be adjusted.

Here, the interval adjustment plates are preferably formed on the bottom surface of the fixing holder so as to overlap each other by rotation.

As shown in FIG. 5D, multiple catching protrusions are formed on the inner surface of the arc-shaped guide hole 332 so that the interval adjustment pin 39 is caught in multiple stages within the arc-shaped guide hole to allow position fixation.

As such, every user has a different interval between two nostrils and thus the adjustment of the angle between the first fixing holder 30a and the second fixing holder 30b may improve sealing properties for nostrils by adjusting an interval between the adapters.

In addition, when the coupling elements 251a, 251b of the first injector 20a and the second injector 20b are not positioned at the axial center of the injector but biased to one side, an interval between inlets/outlets 41a, 41b of the first adapter 40a and the second adapter 40b may be adjusted by rotating injector bodies while coupled to the fixing holders and adjusting an interval between coupling elements of the injectors.

FIG. 6A to FIG. 6D are views illustrating a clip-type fixing holder according to an embodiment of the disclosure.

Referring to the drawings, the fixing holder 30 includes: a clip body 35 having a mounting groove 351 configured to allow the first injector and the second injector mounted thereto, respectively and formed at both lateral sides thereof; clip parts 36a, 36b respectively protruding toward both lateral sides from both ends in the axial direction of the clip body and configured to hold each of the front end and rear end of the first injector and the second injector.

That is, the clip body 35 includes the mounting grooves 351 formed at both lateral sides in the axial direction, and the injectors are mounted so that lateral sides thereof are brought into contact with the mounting grooves. In addition, a pair of clip parts 36a, 36b protrude from each of opposite sides of the axial front end and rear end of the clip body in a lateral direction perpendicular to the axis for forming the mounting grooves.

Each of the pair of clip parts 36a, 36b may be formed in a form of a pair of hooks to surround a portion of the outer surface of the injectors to be mounted and correspond to mounting grooves to improve holding force of the injector and facilitate removal. One or multiple clip parts 36a, 36b may be additionally formed at intermediate positions in addition to the front end and the rear end of the lateral surface of the clip body, or the clip parts may protrude from the whole lateral side to be long clip parts. The clip parts protruding from opposite sides of the mounting grooves may be formed so that both sides are formed to protrude equally or formed to have an asymmetrical length to be used. That is, according to a form of the injector used, the protrusion length may be variously formed. In addition, an internal diameter formed by an inner curved surface of the clip part and the mounting groove is formed to correspond to the size of the injector to be used. That is, when an injector having a large capacity is used, the internal diameter of the clip part and the mounting groove may be formed to be large, and when an injector having a small capacity is used, the internal diameter of the clip part and the mounting groove may be formed to be small so that the injector is mounted, or it may also be provided in a form of using different injectors, in which a large capacity injector is mounted on one side and a relatively small capacity injector is mounted on the other side.

In addition, a catching projection 37 may be further formed to prevent the injector mounted on the inner surface of the clip part 36a formed at the front end of the fixing holder from being pushed to the front. The body front ends of the mounted first injector and second injector are caught by the catching projection 37 and thus the first injector or the second injector is prevented from being slid in the mounting groove to be pushed toward the front and deviated when the piston push rod is pressurized.

In addition, the fixing holder includes an insertion groove 38 formed on the clip part 36b at the rear end of the clip body in a direction perpendicular to the axis of the mounting groove. The insertion groove 38 may allow the handle part protruding from the body rear end of the first injector or the second injector in the outer direction to be recessed inward so that the handle part is caught by the insertion groove to prevent the first injector or the second injector from being pushed in the forward or backward direction when an external force is applied to the first injector and the second injector in the axial direction.

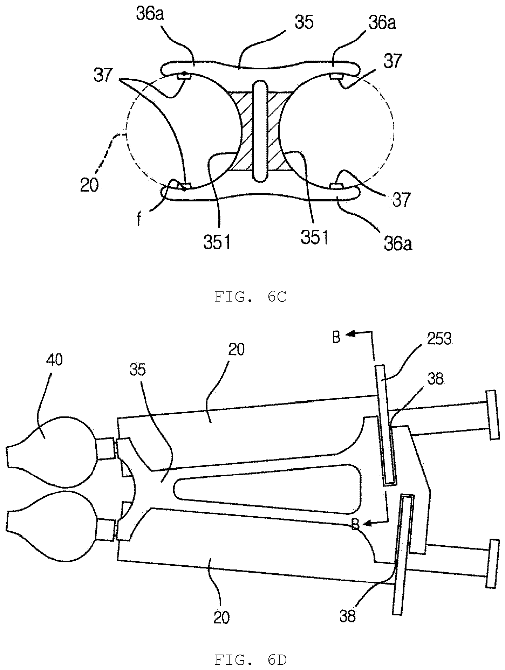

FIG. 6C is a sectional view taken along A-A in FIG. 6B, and as shown in the drawing, front clip parts 36a are formed at opposite sides of the mounting groove 351 formed at opposite sides of the clip body 35, and an L-shaped catching projection is formed inward at the far end of the front clip part 36a to catch the front end of the injector installed therein. In addition, the clip part is formed in a hook shape to contain a curved portion (f: a point at which a straight line passing through the center of the injector is intersected with the outer surface) of the outer surface of the mounted injector so that the injector 20 is prevented from being pushed forward and detached in the lateral direction when mounted to the mounting groove 351.

The insertion grooves formed at the rear clip part 36b of the clip body may be formed on the same line when the first injector and the second injector have the same size, and may be formed to be shifted from each other as shown in FIG. 6A and FIG. 6D when the first injector and the second injector have different sizes. The insertion grooves of opposite sides of the clip part are formed to be shifted to enable the use of the injectors having different capacities and to facilitate handling with one hand by narrowing an interval between the two injectors. Here, the injectors with different capacities are used because each injector forms a different use role. For example, capacity difference may occur in injectors for forming a negative pressure and drug injection. In an embodiment, an injector with 20 ml capacity is used for forming a negative pressure and an injector with 30 ml capacity is used for injecting a drug, or opposite capacities may be used, and it is possible to use them by increasing or decreasing the capacities as necessary.

In addition, the two mounting grooves 351 of the clip body 35 may be formed such that an axial interval of the mounting grooves is parallel or intersected at an acute angle. As such, when the axial interval of the mounting grooves is adjusted, an interval between the inlets/outlets 41a, 41b of the adapter 40a and the adapter 40b mounted at the front end of the injectors 20 to be mounted may be adjusted.

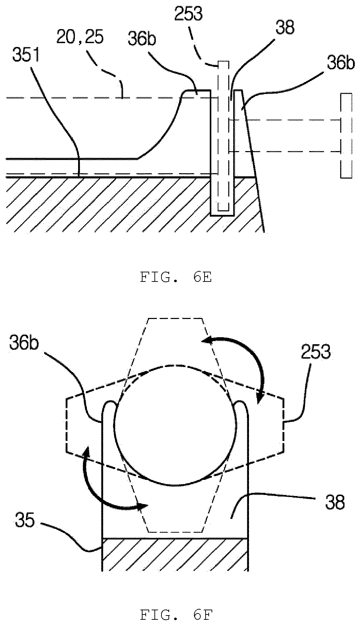

In addition, as shown in FIG. 6E, it is preferable that the insertion grooves 38 are formed in a straight line or a curved shape to have a depth deeper than a protruded length of the handle part 253 of the lateral surface of an injector body when the injector is mounted to the mounting grooves 351. When the insertion grooves are formed to have the depth, the handle part of the injectors may rotate without being caught by the insertion grooves even when the injectors are axially rotated in a state in which the injectors are mounted to the insertion grooves. (See FIG. 6F)

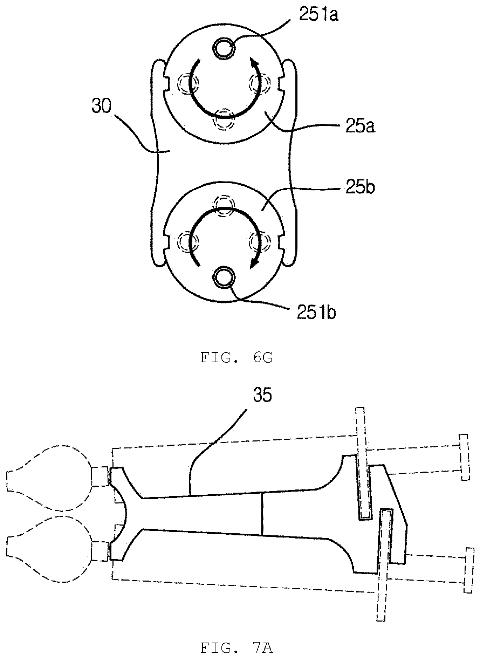

Accordingly, the axial rotation of the injectors mounted to the fixing holder causes the coupling elements 251a, 251b formed at the front end of the syringe bodies 25a, 25b to rotate together. That is, in a case of an injector having a large capacity, the coupling element is position biased to one side from the axial center of the body. Accordingly, when the injector having a biased coupling element is axially rotated, as shown in FIG. 6G, an interval between the coupling elements 251a, 251b formed at two injectors is changed to cause an interval between inlets/outlets of the adapters mounted to each coupling element to be changed, lastly. Therefore, it is possible to adjust the interval between the inlet/outlet of the adapters in accordance with an interval between nostrils of a user, and the nostrils are easily sealed and mucus removal and drug injection are facilitated.

The clip body 35 of the disclosure may be formed to be separated into a front body 35a and a rear body 35b so as to allow length adjustment. That is, the whole length may be adjusted by inserting a portion of the rear body into the separated front body or controlling the degree of insertion thereof.

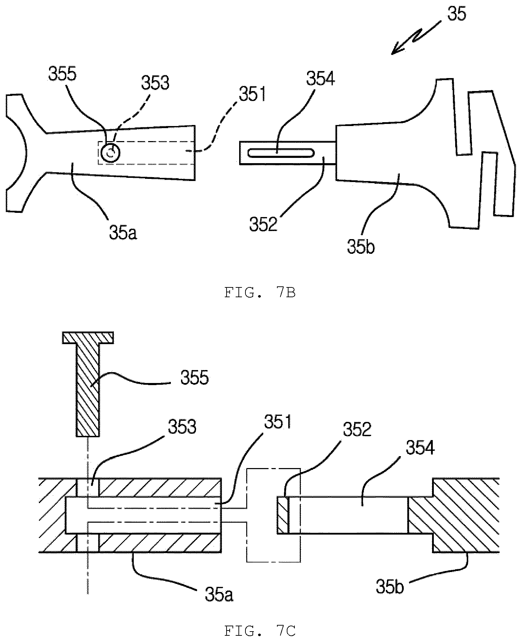

FIG. 7A to FIG. 7C are a planar view and sectional views of main parts, illustrating a clip body including a length adjustment groove and a length adjustment bar formed therein according to the disclosure.

Referring to the drawings, the clip body 35 of the disclosure may be formed to be separated into the front body 35a and the rear body 35b.

A length adjustment groove 351 is formed from the rear end in the front direction inside the front body 35a. The length adjustment groove 351 is a groove which is elongated in the longitudinal direction of the clip body and into which a length adjustment bar 352 to be described below is slidably inserted. A coupling hole 353 is installed in the front body including the length adjustment groove 351 to orthogonally penetrate the length adjustment groove so as to allow a coupling pin 355 to penetrate therethrough.

A length adjustment bar 352 having a section corresponding to a section of the length adjustment groove 351 protrudes from the front end of the rear body 35b in the forward direction. A long coupling hole 354 is formed to penetrate the length adjustment bar 352 in the orthogonal direction and elongated in the longitudinal direction of the long coupling hole 354.

Accordingly, when the length adjustment bar 352 of the rear body is inserted into the length adjustment groove 351 of the front body, the coupling hole 353 of the front body becomes to communicate with the long coupling hole 354 formed through the length adjustment bar, regardless of the insertion depth of the length adjustment bar 352. That is, the coupling pin 355 sequentially penetrates to be coupled to the coupling hole 353 in one lateral wall of the front body, the long coupling hole 354 of the length adjustment bar of the rear body, and the coupling hole 353 in the other lateral wall of the rear body, and may firmly secure the length adjustment bar 352 within the length adjustment groove 351 by tightening. Through the fixing process, the entire length of the clip body 35 may be freely adjusted according to the coupling position of the coupling pin 355 within the long coupling hole 354, and thus the length of the clip body may be adjusted in response to injectors having various lengths.

Here, the coupling pin 355 may be formed of a bolt and fastened by an additionally provided separate nut, or the coupling pin may be coupled by a female thread formed on the inner surface of the coupling hole. In addition, various known coupling means such as hooking method by a hook may be applied in addition to the bolt and nut method.

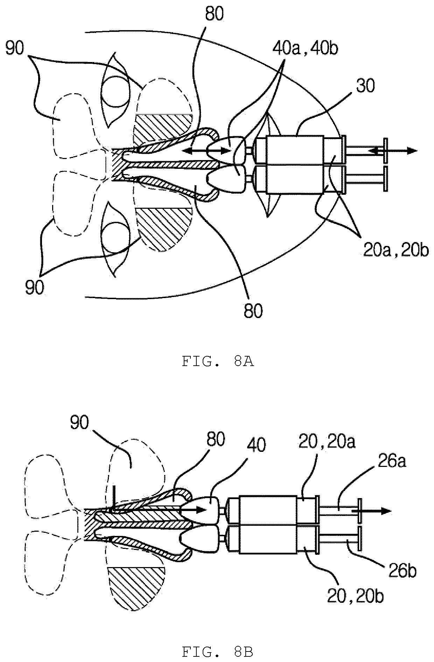

A description of a method of using the dual-body drug administration device having a function of removing mucus from the paranasal sinus of the disclosure, which has the configuration described above will be given. (FIG. 8A to FIG. 8G)

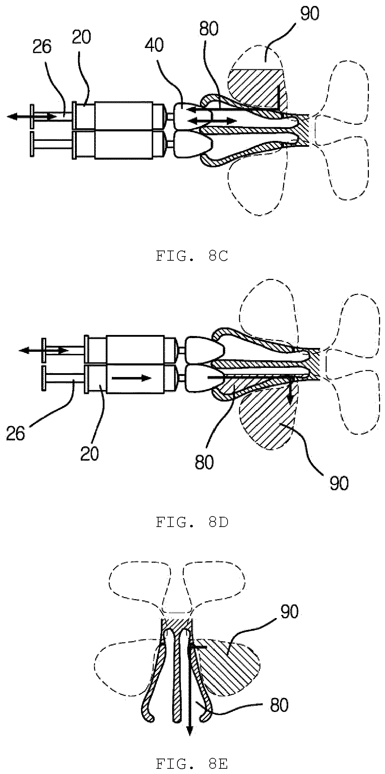

The paranasal sinus 90 is a space connected to the nasal cavity 80 through a very narrow path. The paranasal sinus 90 is a space filled with air and it is thus difficult to inject drugs through the nasal cavity with normal methods. Therefore, the dual-body drug administration device of the disclosure generates a pressure difference to allow mucus and air, or a drug to move between the paranasal sinus and the nasal cavity, and the method thereof is as follows.
1) The first injector 20a and the second injector 20b having the first adapter 40a and the second adapter 40b coupled to front ends thereof, respectively, are seated in and integrally coupled the mounting grooves of the fixing holder 30.
2) In order to make sure that the interval between the inlets/outlets 41a, 41b of each adapter of the first injector and the second injector is suitable for both nostrils, the first injector and the second injector are moved forward/backward in the axial direction, the interval of the interval adjustment part of the fixing holder is adjust, or in case that the injector has a discharging hole biased to one side, the injector itself is axially rotated so that the interval between inlets/outlets 41a, 41b of the adapters is adjusted and fixed to be suitable for the two nostrils.
3) The first adapter and the second adapter of the first injector and the second injector integrally coupled to the fixing holder are brought into close contact with the nostrils of a user.
4) The head is tilted to be horizontal to the side and tilted to locate the paranasal sinus 90, from which mucus is to be removed, at the top.
5) The Mouth is opened, and breathing is performed with the mouth to close the soft palate so as to block nasal breathing.
6) A piston push rod of one of the first injector and the second injector is slowly pulled back. (See FIG. 8A)
7) The air is drained out of the nasal cavity to form a negative pressure throughout the nasal cavity.
8) The pulled piston push rod is pushed so that the air pressure in the nasal cavity is normal.
9) Pushing and pulling a piston push rod of any one injector is repeated several times to allow mucus of the upper paranasal sinus to flow into the nasal cavity.
10) When mucus of the upper paranasal sinus flows into the nasal cavity, the piston push rod of the injector located at the upper end is slowly pulled to collect the mucus into the injector located at the upper end and remove the mucus. In addition, when the upper paranasal sinus is filled with mucus, pushing and pulling the piston of the injector of the lower end is repeated to generate a pressure difference in the upper nasal cavity so that mucus of the upper paranasal sinus additionally flows out to be collected in the upper injector. (See FIG. 8B)
11) The first injector and the second injector are detached from the nose and the received mucus is discarded.
12) The same method is proceeded to remove mucus from the opposite paranasal sinus by tilting the head to the opposite side. (See FIG. 8C)
13) An injector containing a medical fluid is mounted to the fixing holder and brought into close contact with a nostril to cause the adapter to close the nostril to which the drug is to be injected.
14) The head is tilted to be horizontal so that a paranasal sinus into which the drug is to be injected is located at the lower position and the piston push rod of the injector containing the medical fluid and located at the lower position is slowly pushed to fill a lower space of the nasal cavity with the medical fluid. (See FIG. 8D)
15) The mouth is opened and the soft palate is closed to block nasal breathing.
16) Pulling and pushing the piston push rod of the upper injector is slowly repeated to cause the medical fluid of the lower nasal cavity to flow into the lower paranasal sinus from which mucus has been removed. The piston push rod of the lower injector containing the medical fluid is slowly pushed to cause the medical fluid to fill a lower space of the nasal cavity.
17) When the lower paranasal sinus is filled with the medical fluid, the remaining medical fluid filled in the nasal cavity is suctioned and removed, the first injector and the second injector are separated from nostrils, and the medical fluid filled in the paranasal sinus is removed by flowing out by its own weight when the head is located vertically, or paranasal sinus is located at the upper end. (See FIG. 8E). Alternatively, in the same method as the method for removing mucus from the paranasal sinus, pulling the injector push rod in close contact therewith is repeated while the head is tilted to the opposite side to cause the medical fluid of the upper paranasal sinus to flow into the upper injector.

Here, when the pressure difference in the nasal cavity is caused not by the piston push rod of the injector but by the volume change of the injector adapter caused by forward/backward pressurization of the injector, the pressure change in the upper nasal cavity also occurs when the pressure of the lower nasal cavity is changed, and thus mucus may be discharged from the upper paranasal sinus to the upper nasal cavity during a process of administrating a drug from the lower nasal cavity to the lower paranasal sinus.

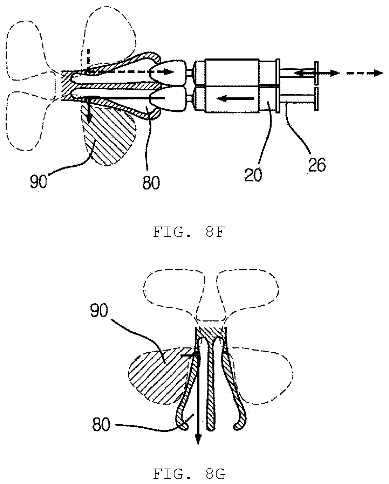

18) The opposite paranasal sinus is treated in the same manner for injecting a drug according to the method. (See FIG. 8F and FIG. 8G)

The method is performed when both paranasal sinuses have mucus therein, and when only one of paranasal sinuses has mucus, a process in which mucus of the one paranasal sinus is removed and a drug is injected may be performed.

In addition, in case that a drug is desired to be injected to a paranasal sinus when there is no mucus in the paranasal sinus, the method may be performed in the same manner to inject the drug into the paranasal sinus.

In addition thereto, a user may execute the method in various ways by selection.

As the drug used, 0.6 to 5 w/v% salt water or a povidone iodine-containing solution having a concentration of 0.01 to 0.3 w/v% may be used. In addition, it may be used for any kind of required liquid medication such as using an antiviral agent-containing solution, an antibiotic or steroid-containing solution, and a vaccine-containing solution.

The method exhibits excellent efficacy in prevention and treatment of rhinitis, allergic rhinitis, sinusitis, chronic sinusitis, a runny nose, a stuffy nose, a cold, a flu, or COVID-19. Particularly, the administration of an iodine drug may achieve an antiinflammatory effect by inhibiting cytokines occurring in an infected area as well as a disinfection effect and thus in addition to eradicating paranasal sinus infectious pathogens, these inflammations and their symptoms may be quickly alleviated or removed.

Furthermore, for the purpose of use, the paranasal sinuses may be cleansed by injecting saline into the paranasal sinuses, Rhinitis, chronic rhinitis, a cold, a flu may be treated by injecting a povidone iodine-containing solution, respiratory viral infection may be prevented and treated by injecting an antiviral agent-containing solution, rhinitis and sinusitis may be prevented and treated by injecting a steroid-containing solution, and immunity may be built by injecting a vaccine-containing solution. In addition thereto, it may be used for injecting various medicines through the nasal cavity.

Specifically, it may exhibit excellent efficacy in eradicating infectious viruses causing infection in the nasal cavity and paranasal sinus, such as rhino virus, corona virus, influenza virus, adenovirus, human para influenza virus, respiratory cell fusion virus, enterovirus other than rhinovirus, metapneumovirus, and MERS and SARS viruses;
if the infection is of bacterial origin, in eradicating Streptococcus pneumoniae, Haemophilus influenzae and Moraxella catarrhalis, the bacterial pathogen that causes sinusitis such as Staphylococcus aureus, and other streptococci species, anaerobes and often gram-negative bacteria; and in improving allergic symptoms caused by dust or pollen.

### Embodiment 1

### Drug administration device 1

The first adapter and the second adapter were mounted to the first injector and the second injector, respectively.

The first injector and the second injector employed a plastic syringe of 20 ml and were integrally coupled by the fixing holder so that an interval between the centers of inlets/outlets formed at the front ends of the first adapter and the second adapter became 20 mm.

### Embodiment 2

### Drug administration device 2

The first adapter and the second adapter were mounted to the first injector and the second injector, respectively.

The first injector employed a syringe of 20 ml and the second injector employed a plastic syringe of 30 ml and they were integrally coupled by the clip type fixing holder so that an interval between the centers of inlets/outlets formed at the front ends of the first adapter and the second adapter became 20 mm.

### Test Example 1-Paranasal Sinus Cleansing

First, the nose was emptied clearly by lightly blowing the nose lightly at the sink, the drug administration device of Embodiment 1 or Embodiment 2 was grabbed with the left hand, all the inner air was pushed out of the injector, and then the device was brought into close contact with two nostrils.

The pressure inside the nasal cavity was repeatedly changed between a negative pressure and a positive pressure through a process of slowly pulling back the piston push rod of the upper injector and pushing it back to the original position while tilting the head to the left to be horizontal, opening the mouth, close the soft palate, and forming lips like whistling to breathe with the mouth.

When mucus flowed out from the upper paranasal sinus while the head is disposed to be horizontal, the mucus is pulled to collect in the upper injector by pulling the upper injector. A negative pressure and a positive pressure are repeatedly applied to the nasal cavity by pushing and pulling the piston push rod of the upper injector until there is no more mucus coming out from the paranasal sinus to the nasal cavity. When mucus is no longer collected, the soft palate is opened, the device is detached from the nose, the collected mucus is discarded, and the injector is washed with running tap water.

A first injector filled with 20 ml of physiological saline and a second injector with empty inside are mounted to the fixing holder, the first injector and the second injector are brought into close contact with the nostrils, respectively, and then the head is tilted to the right to be horizontal so as to allow the nasal cavity to be horizontal. Here, the first injector is disposed in the lower nasal cavity and the stored saline is injected into the nasal cavity by slowly pushing the piston push rod of the first injector to fill the nasal cavity as much as possible while preventing the saline from passing through the throat.

In this state, breathing is performed with the mouth while the soft palate is closed and nasal breathing is blocked, and the pressure in the lower nasal cavity is repeatedly changed by pulling back or pushing the piston push rod of the injector located at the top. Here, when it feels like that saline of the lower nasal cavity is running out, saline is continuously provided to the lower nasal cavity by pushing the piston push rod of the lower injector containing saline little by little.

After saline provision, the pressure changes are repeated in the lower nasal cavity. Here, if the saline comes over to the upper injector or only air reciprocates when the piston push rod of the injector located at the bottom is pushed or pulled, all saline has been injected into the paranasal sinus. The soft palate is opened, the device is detached from the nose, and the injector is washed with running tap water.

The device from which all the inner air has been pushed out is brought into close contact with the nostrils again, the head is tilted to the left side to be horizontal, the soft palate is closed, breathing is performed with the mouth, and a negative pressure and a positive pressure are repeatedly generated in the nasal cavity by slowly pushing and pulling the piston push rod of the injector located at the top.

If when fluid flows out from the upper paranasal sinus, the fluid is collected by slowly pulling the piston push rod of the upper injector.

Pressure changes are repeatedly applied to the nasal cavity by pushing and pulling the piston push rod of the upper injector until there is no more fluid collected into the upper injector. When fluid is no longer collected, the soft palate is opened, the device is detached from the nose, the collected fluid is discarded, and lightly nose blowing is performed.

The injector is washed with running tap water.

The opposite paranasal sinus is cleansed by identically repeating the method.

### Test Example 2-Paranasal Sinus sterilization

### The paranasal sinus is disinfected as in Test

### Example 1 using a saline solution containing 0.2w/v% povidone-iodine.

### Test Example 3-Treatment of Rhinitis

Treatment was performed as in Test Example 1 using a solution containing 0.2w/v% povidone iodine and 2.0w/v% sodium chloride, and 10 adult males and females with rhinitis were treated twice a day for two days, confirming that symptoms were significantly alleviated.

### Test Example 4-Treatment of Cold

Treatment was performed as in Test Example 1 using a solution containing 0.2w/v% povidone iodine and 2.2w/v% sodium chloride, and 10 adult males and females with a cold were treated twice a day for two days, confirming that symptoms were significantly alleviated.

### Test Example 5-Treatment of Flu

Treatment was performed as in Test Example 1 using a solution containing 0.2w/v% povidone iodine and 2.4w/v% sodium chloride, and 10 adult males and females infected with influenza were treated twice a day for two days, confirming that symptoms were significantly alleviated.

### Test Example 6

The same test was performed using the clip type dual-body drug administration device and the same effect was confirmed.

Therefore, it can be verified that the dual body drug administration device of the disclosure is effective in preventing and treating a cold and a flu, nasal secretion, nasal congestion, facial stasis, pain/pressure on the face, loss of smell, and fever which are main characteristics of sinusitis, and symptoms such as a headache, pain/pressure on protrusion, bad breath, dental pain, and fatigue.

### [Brief Description of Reference Numerals]

10: drug administration device
20: injector
20a: first injector 20b: second injector
25, 25a, 25b: body 26, 26a, 26b: piston push rod
251a, 251b: coupling element 252a, 252b: space part
253: handle part
30: fixing holder
30a: first fixing holder 30b: second fixing holder
31: holder body 32: holder cover
33, 33a, 33b: interval adjustment part 34: hinge pin
35: clip body 35a: front body
35b: rear body 36a,36b: clip part
37: catching projection 38: insertion groove
39: interval adjustment pin
311, 351: mounting groove 331a, 331b: hinge hole
332: arc-shaped guide hole 332a: catching protrusion
333: fixing hole
351: length adjustment groove 352: length adjustment bar
353: coupling hole 354: long coupling hole
355: coupling pin
40, 40c: adapter
40a: first adapter 40b: second adapter
41, 41a, 41b, 41c: inlet/outlet 42: front adapter part
43: rear adapter part 44: fluid channel
411: communication groove
80: nasal cavity
90: paranasal sinus

## Claims

1. A dual-body drug administration device for injecting a drug into a nasal cavity through a nostril, the dual-body drug administration device comprising:
a) a first injector (20a) having a coupling element (251a), which is formed at the front end thereof and to which a first adapter (40a) is coupled, and a space part (252a) formed therein;
b) a second injector (20b) having a coupling element (251b), which is formed at the front end thereof and to which a second adapter (40b) is coupled, and a space part (252b) formed therein; and
c) a fixing holder (30) configured to mount and fix the first injector and the second injector such that a distance between centers of an inlet/outlet (41a) of the first adapter (40a) and an inlet/outlet (41b) of the second adapter (40b) is 0.5 cm to 10 cm.

2. The dual-body drug administration device of claim 1,
wherein the fixing holder (30) is configured to:
allow the first injector (20a) and the second injector (20b) to be mounted in parallel so as to be in close contact with a user's both nostrils; or
allow the first injector (20a) and the second injector (20b) to be mounted at an acute angle in a slant state in which an interval between the rear sides thereof is wider than that between the front sides thereof so that an interval between the inlets/outlets (41a, 41b) of the first adapter (40a) and the second adapter (40b) is adjusted to be brought into close contact with and close the user's both nostrils.

3. The dual-body drug administration device of claim 2,
wherein the fixing holder (30) is provided
in a case form configured to cover to receive bodies of the first injector and the second injector, or
in a clip form to which the first injector and the second injector are inserted into opposite lateral sides to be fixed.

4. The dual-body drug administration device of claim 3,
wherein the fixing holder (30) is provided
in a case form so as to comprises: a holder body (31) having mounting grooves (311) configured to allow the first injector and the second injector mounted thereto, respectively; and
a holder cover (32) configured to hold and fix the mounted first injector and second injector by covering the mounting grooves of the holder body.

5. The dual-body drug administration device of claim 4,
wherein the fixing holder (30) further comprises:
interval adjustment parts between two mounting grooves of the holder body (31), and
wherein: the interval adjustment parts (33a, 33b) formed between the mounting grooves are configured to be separate and disposed to overlap each other; on the front side, the separate interval adjustment parts (33a, 33b) are rotatably connected by a hinge pin (34); and
in the rear side, an arc-shaped guide hole (332) having multiple catching protrusions (332a) formed therein is formed through one interval adjustment part (33a) side, a fixing hole (333) is formed through the other interval adjustment part (33b) side, an interval adjustment pin (39) penetrates to connect the arc-shaped guide hole and the fixing hole, and an angle between two mounting grooves is adjusted by left/right rotation of two interval adjustment parts (33a, 33b) which are fixed by tightening the interval adjustment pin (39).

6. The dual-body drug administration device of claim 3,
wherein the fixing holder (30) comprises:
a clip body (35) having mounting grooves (351) configured to allow the first injector and the second injector mounted thereto; and
clip parts (36a, 36b) respectively protruding toward both lateral sides from both ends in the axial direction of the clip body and configured to hold each of the front end and rear end of the first injector and the second injector to be prevented from deviating toward the lateral sides.

7. The dual-body drug administration device of claim 6,
wherein the fixing holder (30) further comprises:
in the clip part 36a of the front end of the clip body (35), a catching projection (37) protruding in a direction of the center axis of the mounting grooves to prevent the mounted first injector and second injector from being pushed to the front by catching the front ends thereof; and
in the clip part (36b) of the rear end of the clip body (35), an insertion groove (38) formed in a direction perpendicular to the center axis of the mounting grooves to allow handle parts formed at the rear ends of the first injector and the second injector to be inserted and fitted thereto.

8. The dual-body drug administration device of claim 7,
wherein a depth of the insertion groove (38) is formed deeper toward the inside from a surface of the mounting grooves to allow a protruding handle part of an injector mounted to the mounting grooves to axially rotate without interruption.

9. The dual-body drug administration device of claim 7,
wherein the clip body (35) is configured to be separated into a front body (35a) and a rear body (35b) to allow length adjustment.

10. The dual-body drug administration device of claim 9,
wherein in the front body (35a), a length adjustment groove (351) is formed from the rear end toward the front side and a coupling hole (353) is formed in a lateral direction orthogonal to the length adjustment groove to be coupled to allow a coupling pin (355) to penetrate therethrough; and
on the rear body (35b), a length adjustment bar (352) having a section corresponding to a section of the length adjustment groove (351) protrudes from the front end in the forward direction to be inserted into the length adjustment groove (351), a long coupling hole (354) is formed inside the length adjustment bar to penetrate in a lateral direction and elongated in the forward/backward direction to allow the coupling pin (355) to penetrate therethrough so that a length is adjusted according to a coupling position of the long coupling hole (354) and the coupling pin (355).

11. The dual-body drug administration device of claim 1,
wherein a negative pressure is generated in the nasal cavity by forward/backward movement of a push rod of the first injector or the second injector to allow mucus inside the paranasal sinus to be discharged or a drug to be injected into the paranasal sinus.

12. A fixing holder as a device included in the dual-body drug administration device according to one of claim 1 to claim 11,
wherein the fixing holder allows two injectors to be mounted and fixed thereto.

13. An adapter as a device included in the dual-body drug administration device according to one of claim 1 to claim 11,
wherein the adapter has a communication groove (411) formed at the end surface of an inlet/outlet thereof in a lateral direction such that internal and external portions communicate with each other.
